# EUROPEAN PATENT APPLICATION

(11) **EP 1 859 747 A1**
(43) Date of publication of application: **28.11.2007**
(21) Application number: 06712828.0
(22) Date of filing: 01.02.2006
(51) Int. Cl.: A61B 17/12, A61B 1/00

(54) **SUTURING AND LIGATING DEVICE FOR MEDICAL TREATMENT**

(30) Priority: 17.03.2005 JP 2005076950
(71) Applicant: Olympus Corporation, Tokyo 151-0072 (JP)
(72) Inventor: MIYAMOTO, Satoshi, Nishitama-gun (JP); ONISHI, Norio, Tokyo (JP)
(74) Representative: von Hellfeld, Axel
(86) International application number: PCT/JP2006/301685
(87) International publication number: WO 2006/098090

(57) **Abstract**

A medical suturing and ligating apparatus is provided which enable to cut a suturing and ligating member such as a ligating wire in a simple manner while preventing a holding member from being removed from a flexible sheath. When a slider 32 pulls a ligating wire 18 toward the proximal end of an inner sheath 7 so as to suture or ligate a living tissue with the ligating wire 18 when a wire holding member is in abutting contact with the distal end of the inner sheath 7, a fixing screw 40 engages with an engaging recessed portion 41. As a result, the slider 32 is fixedly held on an operating section body 31. Accordingly, it is possible to maintain the state in which the wire holding member is in abutting contact with the distal end of the inner sheath.

## Description

### TECHNICAL FIELD

The present invention relates to a medical suturing and ligating apparatus for suturing and ligating living tissues by inserting the apparatus into a body in a transendoscopic manner.

This application is based upon and claims the benefit of priority from the prior Japanese Patent Application No. 2005-76950, filed March 17, 2005, the entire contents of which are incorporated herein by reference.

### BACKGROUND ART

Conventionally, medical suturing and ligating apparatuses for suturing and ligating a pathological lesion portion B of a living tissue, such as a polyp by inserting the apparatuses through an endoscope channel and inserting them into body cavities in a transendoscopic manner are known, for example, from Patent Document 1.

As shown in FIG. 1, the medical suturing and ligating apparatus described in Patent Document 1 includes: an inner sheath (flexible sheath) 100 that is inserted through an endoscope channel; an operating wire 101 that is inserted through the inner sheath 100 so as to extend and retract; a loop shaped ligating wire 102 that is removably connected to the distal end of the operating wire 101; a stopper 103 that is disposed at the proximal end of the ligating wire 102; a holding member 104 that makes contact with the distal end of the inner sheath 100 and holds the ligating wire 102 with a portion of the ligating wire 102 being exposed to the outside; and a cutter 105 which is disposed on an outer periphery on the distal end side of the inner sheath 100 so as to extend and retract.

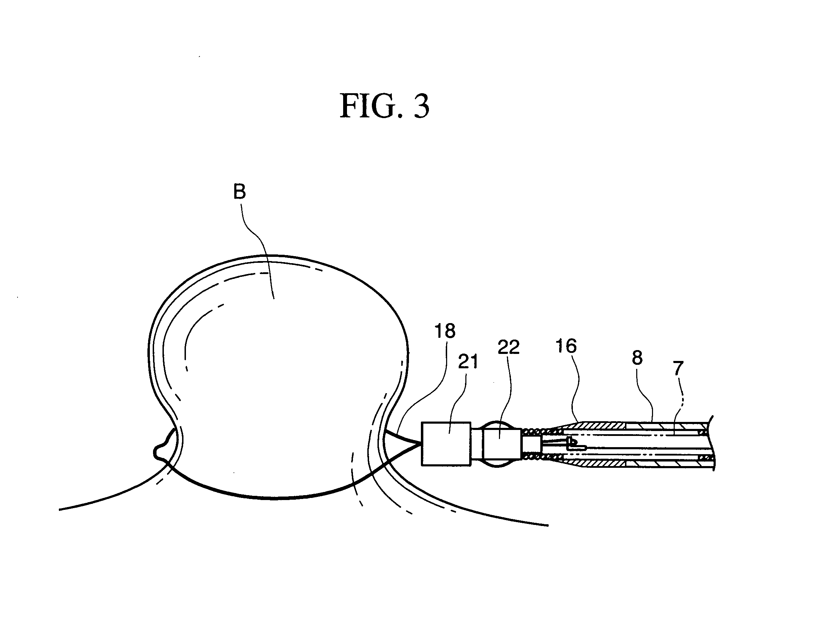

In the medical suturing and ligating apparatus, as shown in FIG. 3, the ligating wire 102 is hooked on the pathological lesion portion B of a living tissue such as a polyp, and the operating wire 101 is pulled toward a handle operating section by operating a wire operating section (not shown). Accordingly, the stopper 103 is moved relatively toward the distal end of the ligating wire 102. As a result, the diameter of the loop of the ligating wire 102 is reduced and thus the pathological lesion portion B is tightly bound.

Thereafter, the cutter 105 is moved toward the distal end of the inner sheath 100 by operating a cutter operating section (not shown) so as to cut the ligating wire 102 that is exposed from the holding member 104 to the outside. Then, the inner sheath 100 and the like are drawn out of the body cavity along with the endoscope.

Since the ligation of the ligating wire 102 stops the blood flow to the pathological lesion portion B, the lesioned tissues necrose and fall off several days later when left in the ligated state. At the same time, the ligating wire 102 and the stopper 103 fall off and are then naturally discharged from the anus. When the inner sheath 100 is drawn out of the body cavity, the holding member 104 escapes out of the inner sheath 100 and is then naturally discharged from the anus.

Patent Document 1: Japanese Unexamined Patent Publication, First Publication No. 2003-204966

### DISCLOSURE OF THE INVENTION

The aforementioned conventional medical suturing and ligating apparatus has several problems as follows.

When the cutter 105 is moved to press and cut the ligating wire 102, the ligating wire 102 may be moved in the moving direction of the cutter 105 by the pressing force of the cutter 105. Accordingly, it is difficult to cut the ligating wire 102 in an appropriate manner. The holding member 104, which is fitted to the distal end of the inner sheath 100 in advance, may be moved in the moving direction of the cutter 105 along with the ligating wire 102 by the pressing force of the cutter 105, and may be disengaged from the inner sheath 100, thereby being removed from the inner sheath 100.

To prevent such a problem, it is necessary to suppress the movement of the ligating wire 102. Moreover, it is necessary to perform a cutting operation of the cutter operating section while a predetermined tension is applied to the operating wire 101 in a direction toward the handle operating section. That is, it is necessary to perform the cutting operation while the operating wire 101 is pulled out toward the handle operating section by the wire operating section. In this case, an operator has to operate the cutter operating section while holding both the wire operating section and an operating section body. Accordingly, the operation becomes complicated.

An object of the invention is to provide a medical suturing and ligating apparatus able to cut a suturing and ligating member such as a ligating wire in a simple manner while preventing a holding member from being removed from a flexible sheath.

According to an aspect of the invention, a medical suturing and ligating apparatus is provided including: a medical suturing and ligating device including: a suturing and ligating member that is made of a flexible wire and is used for suturing and ligating a living tissue; and a holding member that holds the suturing and ligating member while a portion of the suturing and ligating member being exposed to the outside; a flexible sheath to which a proximal end of the suturing and ligating member is inserted; a cutting member that is provided so as to extend and retract in an axial direction of the flexible sheath and is operable to cut the portion of the suturing and ligating member exposed to the outside from the holding member disposed on the distal end of the flexible sheath when the cutting member is moved to the distal end of the flexible sheath; an operating section body that is connected to a proximal end of the flexible sheath; a cutting operating section that is disposed in the vicinity of the operating section body and is connected to the cutting member so as to operate the movement of the cutting member; a suturing and ligating operating section that is disposed in the vicinity of the operating section body and is connected to the suturing and ligating member so as to operate the suturing and ligating member; and a sutured or ligated state maintaining device for maintaining a state in which the holding member is in abutting contact with a distal end of the flexible sheath while the suturing and ligating operating section pulls the suturing and ligating member toward the proximal end of the flexible sheath so as to suture or ligate the living tissue with the suturing and ligating member.

According to the medical suturing and ligating apparatus of the invention, when the suturing and ligating operating section pulls the suturing and ligating member toward the proximal end of the flexible sheath so as to suture or ligate the living tissue with the suturing and ligating member, the sutured or ligated state maintaining device fixes the suturing and ligating member. Accordingly, it is possible to maintain the state in which the holding member is in abutting contact with the distal end of the flexible sheath. Therefore, when the cutting operating section moves the cutting member toward the distal end of the flexible sheath so as to cut the suturing and ligating member, it is possible to prevent the holding member from moving in the moving direction of the cutting member along with the suturing and ligating member by being pressed by the cutting member. Accordingly, it is possible to prevent the holding member from escaping from the distal end of the flexible sheath in the course of the cutting operation. Also, it is possible to cut the suturing and ligating member between the flexible sheath and the holding member in a reliable manner because the holding member is immovable.

In the medical suturing and ligating apparatus, the sutured or ligated state maintaining device may include a fixing device for fixing the suturing and ligating operating section at an arbitrary or specific position relative to the operating section body.

In this case, since the position of the suturing and ligating operating section can be fixed relative to the operating section body, it is thus possible to fix the positions of the holding member and the suturing and ligating member relative to the inner sheath. Accordingly, it is possible to prevent the holding member from escaping from the distal end of the flexible sheath in the course of the cutting operation. Also, it is possible to cut the suturing and ligating member while applying a tension required to the suturing and ligating member and thus to be cut in a reliable manner.

In the medical suturing and ligating apparatus, the fixing device may include: a screw that is provided to one of the suturing and ligating operating section and the operating section body; and an engaging recessed portion that is provided to the other of the suturing and ligating operating section and the operating section body so as to engage the distal end of the screw.

In this case, when the suturing and ligating operating section is moved to a predetermined position relative to the operating section body, the distal end of the screw engages with the engaging recessed portion by means of the screw. Accordingly, it is possible to fix the position of the suturing and ligating operating section relative to the operating section body.

In the medical suturing and ligating apparatus, the fixing device may include: a pin that is provided to one of the suturing and ligating operating section and the operating section body so as to be biased toward the other of the suturing and ligating operating section and the operating section body by a biasing member; and an engaging recessed portion that is provided to the other of the suturing and ligating operating section and the operating section body so as to engage the distal end of the pin.

In this case, when the suturing and ligating operating section is moved to a predetermined position relative to the operating section body, the distal end of the pin engages with the engaging recessed portion by the mechanical movement of the pin caused by the biasing force of the biasing member. Accordingly, it is possible to fix the position of the suturing and ligating operating section relative to the operating section body.

In the medical suturing and ligating apparatus, the fixing device may include: a ratchet plate that is provided to one of the suturing and ligating operating section and the operating section body; and a ratchet claw that is provided to the other of the suturing and ligating operating section and the operating section body so as to engage the ratchet plate and thus to allow a unidirectional movement of the ratchet plate.

In this case, whenever the suturing and ligating operating section is moved in one direction relative to the operating section body, for example, in the direction in which the loop on the distal end of the ligating member is reduced in diameter, the ratchet plate engages with the ratchet claw. Accordingly, it is possible to fix the position of the suturing and ligating operating section relative to the operating section body.

### Advantageous Effect of the Invention

According to the medical suturing and ligating apparatus of the invention, it is possible to maintain the state in which the holding member is in abutting contact with the distal end of the flexible sheath by means of the sutured or ligated state maintaining device. Therefore, when the cutting operating section moves the cutting member toward the distal end of the flexible sheath so as to cut the suturing and ligating member, it is possible to prevent the holding member from moving in the moving direction of the cutting member along with the suturing and ligating member by being pressed by the cutting member. Accordingly, it is possible to prevent the holding member from escaping from the distal end of the flexible sheath in the course of the cutting operation. Also, it is possible to cut the suturing and ligating member between the flexible sheath and the holding member in a reliable manner because the holding member is in abutting contact with the flexible sheath and thus is made immovable.

### BRIEF DESCRIPTION OF THE DRAWINGS

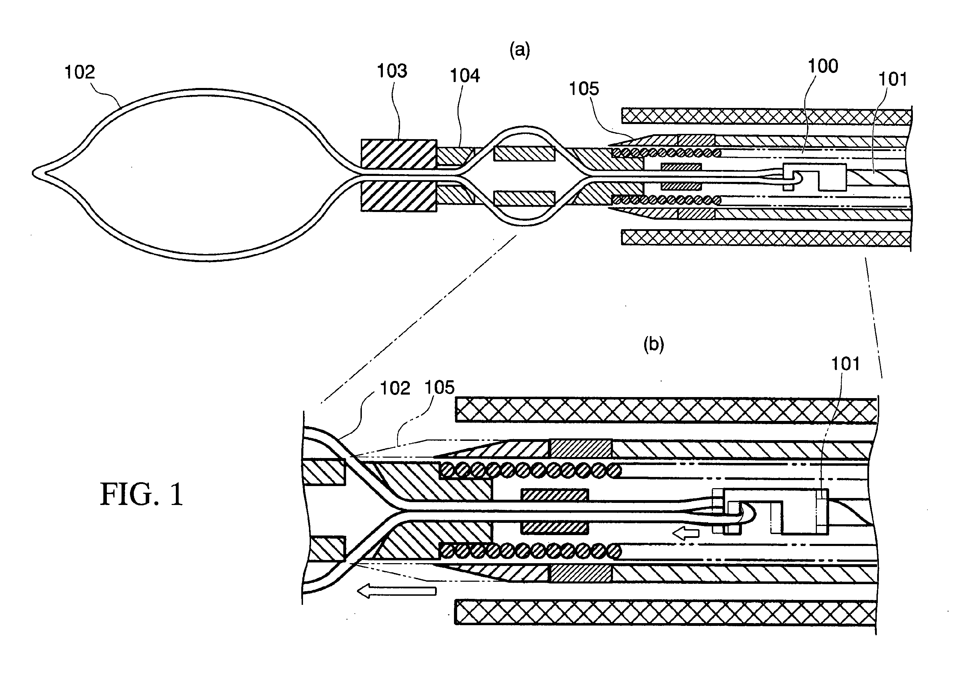

FIG. 1 is a sectional view for explaining conventional problems.
FIG. 2 is a side view for explaining the structure of a medical suturing and ligating apparatus according to a first embodiment, in which a main part thereof is shown in a sectional view.
FIG. 3 is a perspective view for explaining a treatment method using the medical suturing and ligating apparatus of the first embodiment.
FIG. 4 is a side view for explaining a treatment method using the medical suturing and ligating apparatus of the first embodiment, in which a part of a handle operating section is shown in a sectional view.
FIG. 5 is a view for explaining a treatment method using the medical suturing and ligating apparatus of the first embodiment, in which an insertion section is shown in a sectional view.
FIG. 6 is a perspective view for explaining a treatment method using the medical suturing and ligating apparatus of the first embodiment.
FIG. 7 is a side view for explaining the structure of a medical suturing and ligating apparatus according to a second embodiment, in which a main part thereof is shown in a sectional view.
FIG. 8 is a side view for explaining a treatment method using the medical suturing and ligating apparatus of the second embodiment, in which a part of a handle operating section is shown in a sectional view.
FIG. 9 is a perspective view for explaining the structure of a medical suturing and ligating apparatus according to a third embodiment, showing a main part thereof.
FIG. 10 is a perspective view for explaining the structure of the medical suturing and ligating apparatus of the third embodiment, in which a main part thereof is shown in a partial sectional view.
FIG. 11 is a sectional view for explaining the structure of the medical suturing and ligating apparatus of the third embodiment, taken along the line A-A of FIG. 9.

### DESCRIPTION OF REFERENCE NUMERALS AND SIGNS

- 1:: medical suturing and ligating apparatus
- 2:: medical ligating device (medical suturing and ligating device)
- 3:: manipulation device
- 4:: insertion section
- 5:: handle operating section
- 7:: inner sheath (flexible sheath)
- 16:: cutting blade (cutting member)
- 22:: wire holding member (holding member)
- 31:: operating section body
- 31 a:: finger hook ring
- 32:: slider (suturing and ligating operating section)
- 33:: cutting operating section
- 40:: fixing screw (screw, fixing device)
- 41:: engaging recessed section (fixing device)
- 50:: fixing pin (fixing device)
- 66:: ratchet claw (fixing device)
- 68:: ratchet portion

### BEST MODE FOR CARRYING OUT THE INVENTION

Hereinafter, a medical suturing and ligating apparatus according to embodiments of the invention will be described with reference to the accompanying drawings. However, the invention is not limited to the following embodiments, but, for example, elements of the embodiments may be combined in an appropriate manner.

### First Embodiment

First, a medical suturing and ligating apparatus according to a first embodiment of the invention will be described with reference to the accompanying drawings.

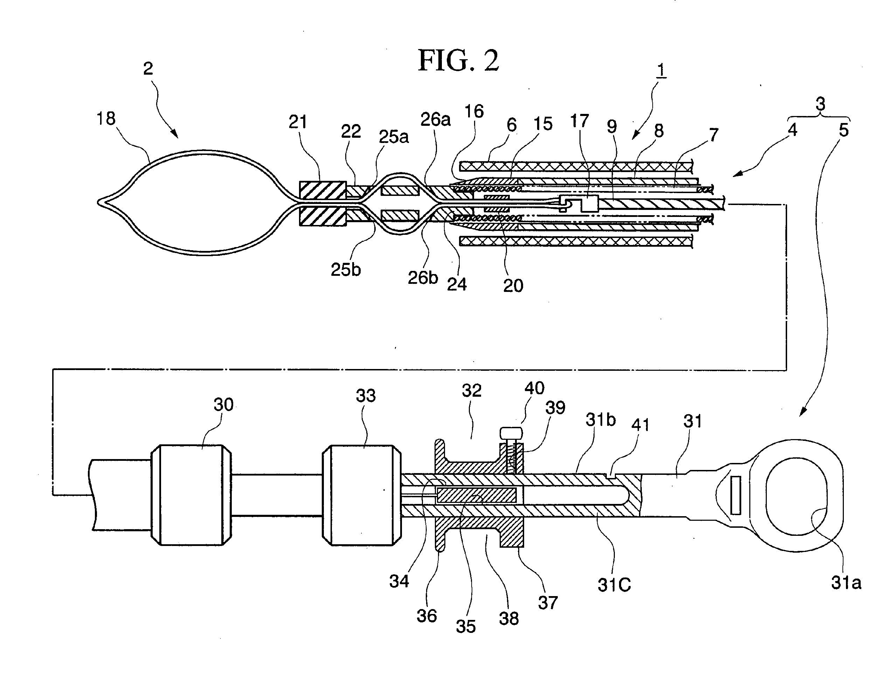

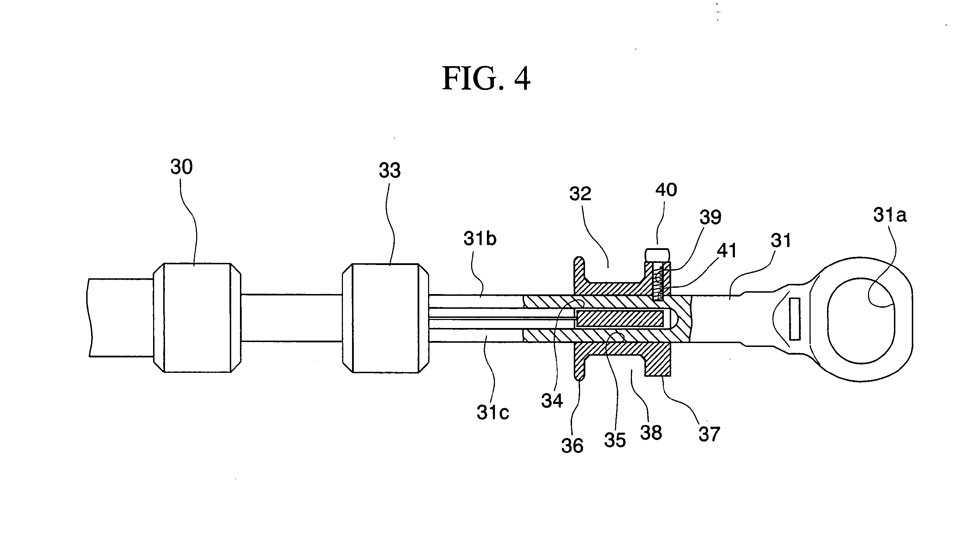

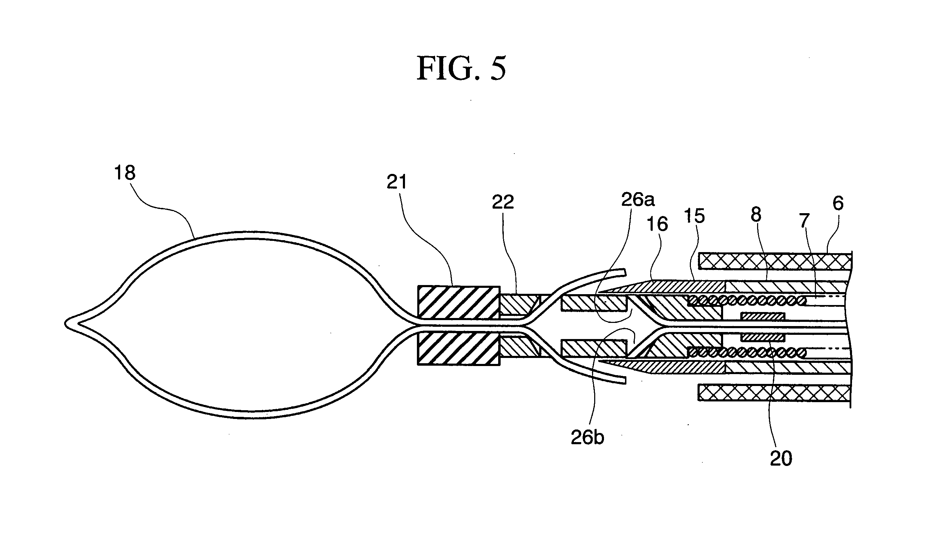

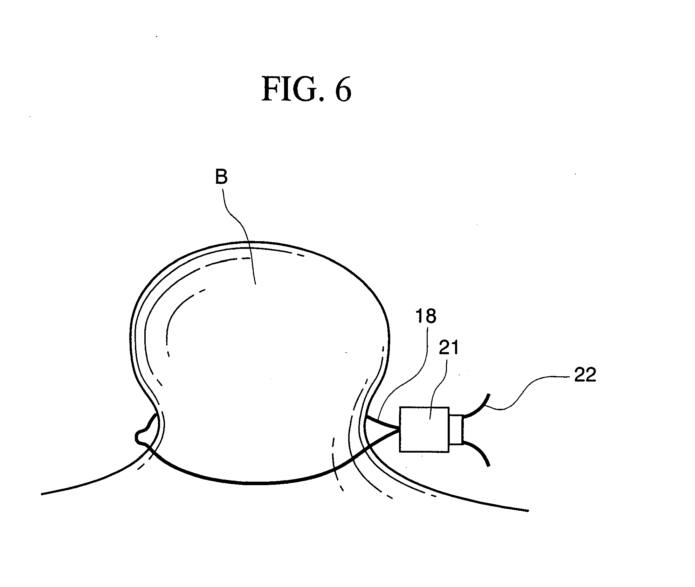

FIGS. 2 to 6 show the first embodiment of the invention. FIG. 2 is a view showing the entire configuration of the medical suturing and ligating apparatus, particularly showing the distal end thereof in a sectional view. FIG. 3 is a perspective view showing a state in which a pathological lesion portion B is ligated with a ligating wire. FIG. 4 is a side view of a handle operating section at that moment. FIG. 5 is a sectional view showing a state in which the ligating wire is cut. FIG. 6 is a perspective view of a pathological lesion portion B at that moment.

As shown in FIG. 2, a medical suturing and ligating apparatus 1 includes a medical ligating device 2 that is retained in a living body and an operation unit 3 that introduces the medical ligating device 2 into the living body so as to perform a ligating operation. The operation unit 3 has a flexible insertion section 4 that is inserted into an endoscope channel and a handle operating section 5.

The insertion section 4 includes an outer sheath 6 that is flexible, an inner sheath 5 that is flexible and is inserted into the outer sheath 6 so as to extend and retract in the axial direction thereof, a cutting sheath 8 that is inserted so as to extend and retract in the axial direction and is fitted onto an outer surface of the inner sheath 7; and an operating wire 7 that is inserted through the inner sheath 7 so as to extend and retract in the axial direction.

The outer sheath 6 is made of, for example, a flexible plastic such as polyethylene and PTFE, and has an outer diameter of 2 to 5 mm, for example. The inner sheath 7 and the cutting sheath 8 are also made of, for example, a flexible plastic such as polyethylene and PTFE, but may additionally include a metal mesh added thereto or may be made of a metal coil. The operating wire 9 is made of a twisted metal wire of stainless steel or the like.

An annular connecting member 15 made of a metallic material such as stainless steel is disposed on the distal end of the cutting sheath 8. A cutting blade (cutting member) 16 forming an acute angle is formed around the distal end of the connecting member 15. An engaging member 17 having a hook shape is fixed to the distal end of the operating wire 9.

Now, the medical ligating device 2 will be described. Reference numeral 18 denotes a loop shaped ligating wire that is used to ligate a living tissue at the distal end thereof. The ligating wire 18 is a thread made of a synthetic resin such as nylon or polyolefin, a fine metal wire of stainless steel or the like, silk, a bioabsorbable material and has a diameter of 0.2 to 1 mm, for example. The ligating wire 18 may be in any form including a single wire, a twisted wire, a woven wire, and the like. In addition, the ligating wire 18 is folded at the proximal end thereof and thus forming a folded portion 19. Both ends of the ligating wire 18 and the parallel two wires are fixed to an internal cavity of a connecting pipe 20 by means of adhesive bonding or the like.

A tube-shaped stopper 21 is fitted to the intermediate portion of the ligating wire 18 so as to extend and retract. The stopper 21 is made of, for example, rubber such as silicon rubber or fluorine rubber or a variety of thermoplastic elastomers, or a knot of a thread. When the stopper 21 is made to extend, a loop portion is reduced in diameter. When the stopper 21 is made to retract, the loop portion is increased in diameter.

A wire holding member 22 for holding the proximal end of the ligating wire 18 in a desirable shape is provided on the ligating wire 18 disposed on the proximal end of the stopper 21. The wire holding member 22 is formed in a cylindrical shape, and is made of a metal such as stainless steel or a plastic such as polypropylene, ABS, polyacetal, or polycarbonate. A diameter-reduced portion 24 is provided at the proximal end of the wire holding 22 and is inserted into the inner sheath 7 so as to be supported on the distal end of the inner sheath 7. Therefore, there is no stepped portion between an outer peripheral face of the inner sheath 7 and an outer peripheral face of the wire holding member 22, and thus allowing smooth extending and retracting of the cutting sheath 8.

A pair of distal lateral holes 25a and 25b through which the ligating wire 18 can be inserted is formed in the distal end of the wire holding member 22. A pair of proximal lateral holes 26a and 26b through which the ligating wire 18 can be inserted is formed in the proximal end of the wire holding member 22. The inner face on the distal end of the distal lateral holes 25a and 25b and the inner face on the proximal end of the proximal lateral holes 26a, 26b are formed on an incline surface. Thus, the ligating wire 18 can be easily inserted into and drawn out of the holes.

The folded portions of the ligating wire 18 are led from a proximal opening of the wire holding member 22 to the inside of the wire holding member 22. Next, the folded portions are led from the proximal lateral holes 26a and 26b to the outside of the wire holding member 22. Then, the folded portions are led from the distal lateral holes 25a and 25b to the inside of the wire holding member 22. Finally, the folded portions are led from the distal opening of the wire holding member 22 to the outside thereof. At the distal end of the wire holding member 22, the ligating wire 18 is pressed into an internal cavity of the stopper 21 in a bound state. Further, the full length of the wire holding member 22 is about 5 mm to 10 mm, for example.

Now, the handle operating section 12 will be described. The handle operating section includes a handle 30 that is fixed to the proximal end of the outer sheath 6; an operating section body 31 that is connected to the proximal end of the inner sheath 7 and is provided with a finger hook ring 31a at the proximal end thereof; a slider 32 that is connected to the proximal end of the operating wire 9 and is disposed in the vicinity of the operating section body 31 so as to extend and retract relative to the operating section body 31; and a cutting operating section 33 that is connected to the proximal end of the cutting sheath 8 and is disposed in the vicinity of the operating section body 31 so as to extend and retract relative to the operating section body 31.

With the handle 30, the slider 32, and the cutting operating section 33, the outer sheath 6, the operating wire 9, and the cutting sheath 8 are relatively movable in the forward and backward directions (between the distal end and the proximal end).

The slider 32 is provided with flanges 36 and 37 at both ends thereof and is substantially formed in a cylindrical shape. Two through-holes 34 and 35 are formed in the central portion of the slider 32 in the axial direction thereof. Bifurcated portions 31 b and 31 c formed in the intermediate portion of the operating section body 31 are inserted through the through-holes 34 and 35, respectively. The slider 32 is guided by the bifurcated portions 31 b and 31 c and is movable in the longitudinal direction. The flanges 36 and 37 are disposed on the distal and proximal ends of the slider 32, respectively, and a ring-shaped groove portion 38 is formed between the distal flange 36 and the proximal flange 37 so as to allow operators to hook their fingers on the groove portion 38.

The distal flange 37 is made thicker than the proximal flange 36. A female screw portion 39 is perforated into the outer peripheral face of the distal flange 37 in a radial direction so as to extend to the through-hole 34. A fixing screw 40 is screwed into the female screw portion 39. An engaging recessed portion 41 is formed in one bifurcated portion 3 1 b of the operating section body 31 to correspond to an inner opening of the female screw portion 39 of the slider 32. The position of the engaging recessed portion 41 is determined in such a manner that the ligating wire 9 in the reduced-diameter state can hold the pathological lesion portion B in the tightly bound state, wherein the reduced-diameter state of the ligating wire 9 is determined based on the positional relation of the engaging recessed portion 41 when engaging with the distal end of the fixing screw 40, relative to the operating section body 31 and the slider 32 and to the inner sheath 7, the operating wire 9, and the like connected thereto.

Here, the fixing screw 40 and the engaging recessed portion 41 constitute the fixing device for fixing the slider 32 at a predetermined position relative to the operating section body 31.

The engaging recessed portion 41 may have a circular shape, a circular cone shape, or a circular truncated cone shape as long as it can engage with the distal end of the fixing screw 40.

Next, the operation of the first embodiment will be described.

First, the ligating wire 18 is inserted into the outer sheath 6, and the loop portion is contracted. In this state, the insertion section 4 is inserted through the endoscope channel and is inserted into a body cavity. Then, the distal end of the insertion section 4 is guided to a target site in the body cavity. When the outer sheath 6 is made to retract by operating the handle 30 while observing the pathological lesion portion B such as a polyp with an endoscope, the ligating wire 18 protrudes from the distal opening of the outer sheath 6, and the loop portion of the ligating wire 18 is elastically restored and is thus increased in diameter, as shown in FIG. 2.

Next, the loop portion of the ligating wire 18 is hooked onto the root of the pathological lesion portion B while observing the pathological lesion portion B with the endoscope, and the operating wire 9 is made to retract by operating the slider 32. Then, the proximal end of the ligating wire 18 is drawn into the inner sheath 7 by means of the engaging member 17. Accordingly, the stopper 21 and the wire holding member 22 are relatively moved toward the distal end of the ligating wire 18 while sliding over the ligating wire 18. Therefore, the loop portion of the ligating wire 18 is reduced in diameter, as shown in FIG. 3, and the pathological lesion portion B is tightly bound. As a result, the blood flow to the pathological lesion portion B is stopped by the ligation.

At this time, the slider 32 is disposed at a position relative to the operating section body 31 in which the distal end of the fixing screw 40 is opposed to the engaging recessed portion 41. In this state, the distal end of the fixing screw 40 is engaged with the engaging recessed portion 41 by fastening the fixing screw 40. Accordingly, it is possible to fix the position of the slider 32 relative to the operating section body 31.

Next, the cutting sheath 8 is made to extend by operating the cutting operating section 33 when the fixing screw 40 is engaged with the engaging recessed portion 41 and the position of the slider 32 is fixed relative to the operating section body 31. Then, the cutting sheath 8 extends while being guided by the inner sheath 7 and thus the cutting blade 16 is also moved in that direction. With the forward movement of the cutting blade 16, the proximal end portion of the ligating wire 18 inserted into the wire holding member 22 is cut in the vicinity of the proximal lateral holes 26a and 26b.

At this time, as described above, since the fixing screw 40 is engaged with the engaging recessed portion 41 and the position of the slider 32 is fixed relative to the operating section body 31, the proximal end portion of the ligating wire 18 is stretched toward the handle operating section 5 by the operating wire 9. Therefore, the wire holding member 22 is stretched toward the handle operating section along with the ligating wire 18 connected to the operating wire 9. Accordingly, it is possible to maintain the state in which the wire holding member 22 is in abutting contact with and is fitted to the distal end of the inner sheath 7. As a result, even when the cutting blade 16 is made to extend by operating the cutting operating section 33 so as to cut the ligating wire, it is possible to prevent the wire holding member 22 from being pressed by the cutting blade 16 and thus moving in the moving direction (the forward direction) of the cutting blade 16. Accordingly, it is possible to prevent the wire holding member 22 from escaping from the distal end of the inner sheath 7.

At the same time, since the ligating wire 18 maintains the state in which it is stretched toward the handle operating section, a predetermined tension is applied to the proximal end of the ligating wire 18. Accordingly, it is possible to cut the ligating wire 18 between the inner sheath 7 and the wire holding member 22 in a reliable manner because the wire holding member 22 is in abutting contact with the distal end of the inner sheath 7 and thus is made immovable.

The two (upper and lower) ligating wires 18 are not cut simultaneously, but may be cut individually. However, since the two ligating wires 20 are coupled to the connecting pipe 20, the tension is maintained even when only one end is cut. Accordingly, the cutting effect is not varied.

When the ligating wire 18 is cut at the proximal end portion thereof, the inner sheath 7 is separated from the wire holding member 22, and the medical ligating device 2 is completely separated from the operation unit 3. When the insertion section 4 is pulled out of the endoscope channel, the wire holding member 22 is made to extend out of the cut proximal end of the ligating wire 18. Accordingly, as shown in FIG. 6, only the ligating wire 18 held in the tightly bound state by the stopper 22 is retained in the body, and the manual operation of tightly binding the pathological lesion portion B is finished.

The wire holding member 22 separated from the ligating wire 18 is naturally discharged from the body through a digestive canal. In this way, according to the medical suturing and ligating apparatus 1, the operation of tightly binding and ligating the pathological lesion portion B with the ligating wire 18 and the operation of cutting and separating the ligating wire 18 can be performed in a series of operations.

In the first embodiment described above, the fixing screw 40 is provided to the slider 32 and the engaging recessed portion 41 is provided to the operating section body 31. However, the fixing screw 40 may be provided to the operating section body 31 and the engaging recessed portion 41 may be provided to the slider 32. In this case, the portion of the operating section body being provided with the fixing screw may be exposed to the outside from the slider.

In addition, in the first embodiment described above, one engaging recessed portion 41 is provided to the operating section body 31. However, the invention is not limited to this, and a plurality of engaging recessed portions may be formed. If the slider 32 can be fixed to the operating section body 31 by means of the fixing force of the fixing screw 40, it is not necessary to form the engaging recessed portion 41. In this case, micro-protrusions or micro-cavities may be formed on the portion of the operating section body 31 making abutting contact with the distal end of the fixing screw 40, for example, on the surface of the bifurcated portion 31b.

### Second Embodiment

Next, a medical suturing and ligating apparatus according to a second embodiment of the invention will be described with reference to the accompanying drawings. The same elements and structures as the first embodiment are denoted by the same reference numerals and repeated descriptions thereof are omitted.

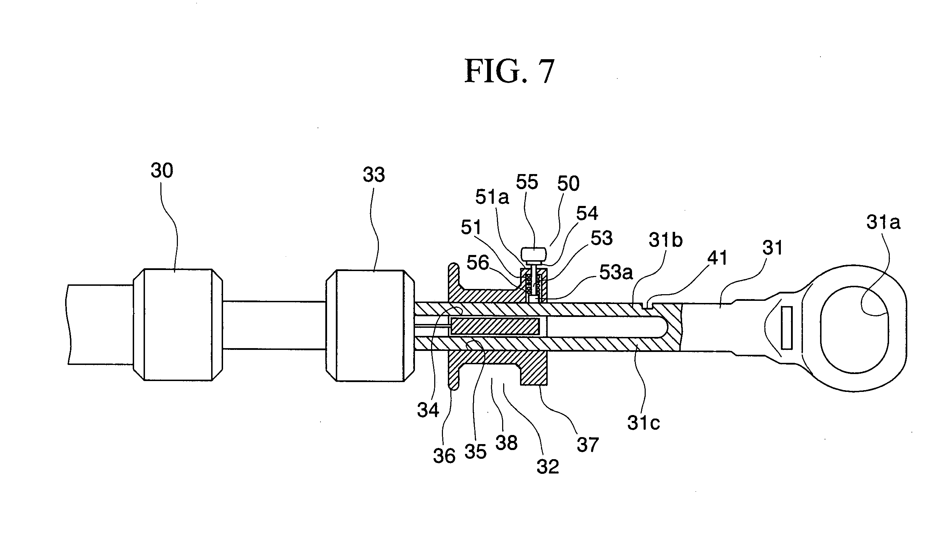

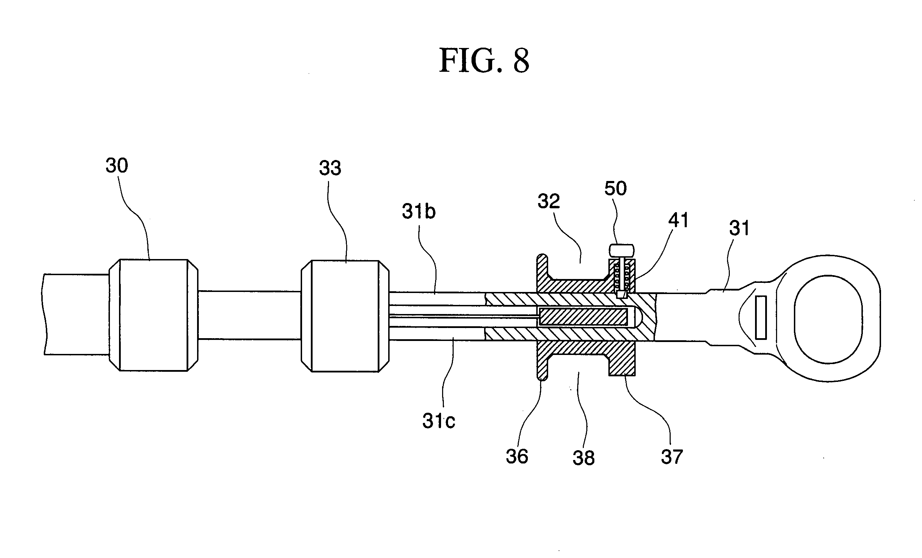

FIGS. 7 and 8 show the second embodiment of the invention, wherein FIG. 7 is a side view of the handle operating section; and FIG. 8 is a side view showing the state in which the slider 32 of the handle operating section is fixed to the operating section body 31.

The second embodiment is different from the first embodiment in that instead of the fixing screw 40, a fixing pin 50 is used to fix the slider 32 to the operating section body 31.

That is, a through-hole 51 is formed in the proximal flange 37 of the slider 32 in a radial direction and extends from the outer peripheral face of the proximal flange 37 to the through-hole 34. The outer peripheral end portion of the through-hole 51 forms a narrow portion 51 a narrower than the inner peripheral end portion, and the narrow portion 51a serves as a spring receiving portion. The distal end of the fixing pin 50 is inserted into the through-hole 51 from above. The fixing pin 50 includes a small-diameter portion 53 that is inserted into the through-hole 51; a large-diameter portion 54 that protrudes out from the through-hole 51; and a grasping portion 55 that is formed in the outer end of the large-diameter portion 54. A spring seat 53a is formed in the inner end of the small-diameter portion 53. When the fixing pin 50 is inserted into the through-hole 51, a compression spring (biasing member) is placed between the spring seat 53a of the fixing pin 50 and the portion on the slider 32 side of the narrow portion 51a. Accordingly, the fixing pin 50 is biased toward the inside of the slider 32.

In the medical suturing and ligating apparatus according to the second embodiment, as shown in FIG. 3, the loop portion of the ligating wire 18 is hooked onto the root of the pathological lesion portion B while observing the pathological lesion portion B with the endoscope, and the operating wire 9 is made to retract by operating the slider 32. At this time, when the fixing pin 50 is moved to a position in which the distal end of the fixing pin 50 is opposed to the engaging recessed portion, the fixing pin 50 is mechanically moved toward the inside of the slider because the fixing pin 50 is biased toward the inside by the compression spring 56 as shown in FIG. 8. Accordingly, the distal end of the fixing pin 50 engages with the engaging recessed portion 41.

That is, in the first embodiment, when an operator engages the slider 32 with the operating section body 31, the operator has to make sure that the fixing screw 40 reaches the engaging recessed portion 41 and then to rotate the fixing screw 40. However, in the second embodiment, it is not necessary to perform such operations. When the slider 32 is moved to a predetermined position, the fixing pin 50 is moved to the inside and engages with the engaging recessed portion 41. Accordingly, the slider 32 engages with the operating section body 31 in a mechanical manner.

Thereafter, in a similar manner to the case of the first embodiment, the cutting sheath is made to extend by operating the cutting operating section and the ligating wire 18 is cut by the cutting blade 16.

In the second embodiment, the slider 32 can be fixed to the operating section body 31 by engaging the fixing pin 50 with the engaging recessed portion 41. Accordingly, it is possible to maintain the state in which the wire holding member 22 is in abutting contact with the distal end of the inner sheath 7 without a need to operate three operating sections at the same time, i.e., without need to operate the slider 32, the operating section body 31, and the cutting operating section 33. Also, it is possible to maintain a predetermined tension applied to the ligating wire 18 until the time of the cutting operation. As a result, it is possible to prevent removal of the wire holding member 22 and thus to cut the ligating wire 18 in a reliable manner.

To disengage the slider 32 from the operating section body 31, it is only necessary to pull out the fixing pin 50 while resisting the force of the compressing spring 56. Thereafter, when the slider 32 is moved toward the distal end, the fixing pin 50 does not engage with the engaging recessed portion 41.

In the second embodiment described above, the fixing pin 50 is provided to the slider 32 and the engaging recessed portion 41 is provided to the operating section body 31. However, the fixing pin 50 may be provided to the operating section body 31 and the engaging recessed portion 41 may be provided to the slider 32.

### Third Embodiment

Next, a medical suturing and ligating apparatus according to a third embodiment of the invention will be described with reference to the accompanying drawings. The same elements and structures as the first embodiment are denoted by the same reference numerals and repeated descriptions thereof are omitted.

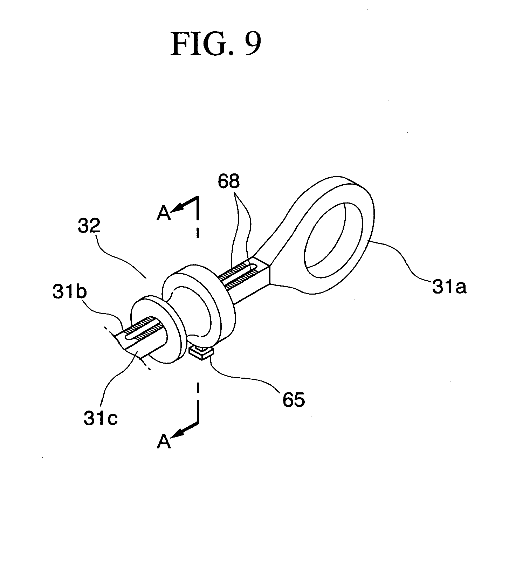

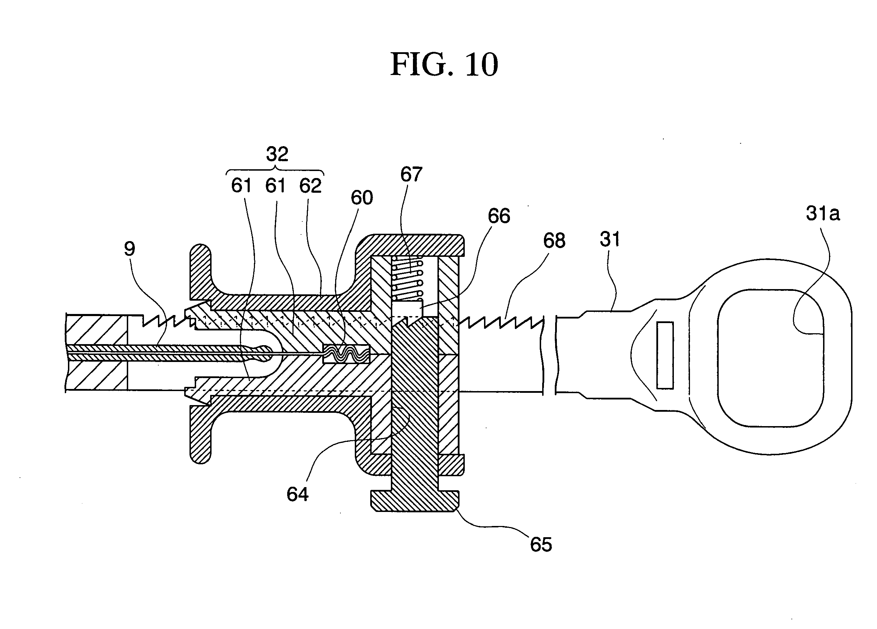

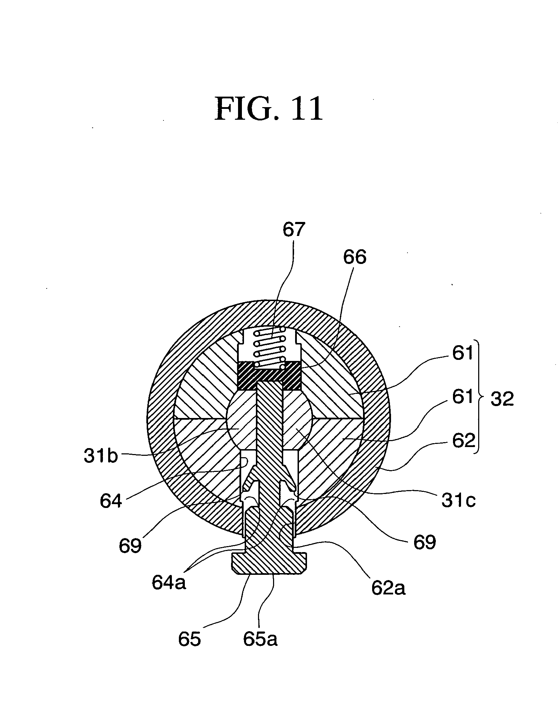

FIGS. 9 to 11 show the third embodiment of the invention, wherein FIG. 9 is a perspective view of the handle operating section; FIG. 10 is a sectional view showing the engaged state of the slider and the operating section body 31; and FIG. 11 is a sectional view taken along the line A-A of FIG. 9.

In the third embodiment, a combination of a ratchet plate and a ratchet claw is used as a fixing device for fixing the slider 32 to the operating section body 31.

As shown in FIGS. 9 and 10, the slider 32 includes, as a main part thereof, two pressing members 61 and 61 configured to fix a wire receiving section 60 attached to the proximal end of the operating wire 9 by interposing them between the two pressing members; and a cover member 62 that covers the entire outer surface of the pressing members 61 and 61.

A rectangular through-hole 64 is formed in large-diameter portions 61 a and 61 a on the proximal end of the pressing members 61 and 61, and a ratchet release button 65, a ratchet claw 66, and a compression spring 67 are disposed in the through-hole 64 so as to be movable in a sliding manner. The compression spring 67 is disposed between the inner walls of the cover member 62 and the ratchet claw 66. Thus, in a normal state, the ratchet claw 66 is pressed toward the operating section body 31. The ratchet portion 68 is formed on a portion of the bifurcated portions 31b and 31c of the operating section body disposed opposite the ratchet claw 66. The bifurcated portion 31b having the ratchet portion 68 formed thereon serves as a ratchet plate. In this way, the ratchet claw 66 is provided to the slider 33 and the ratchet portion 68 is provided to the operating section body 31. Accordingly, the slider 32 is allowed to move toward the proximal end of the operating section body 31, but an opposite directional movement thereof is not allowed.

That is, the ratchet claw 66 and the ratchet portion 68 form a ratchet mechanism.

The ratchet release button 65 passes through the through-hole 64 and a communication hole 62a of the cover member that communicates with the through-hole 64. When the ratchet release button 65 is pressed, the ratchet claw 66 is pushed upward and thus the engagement between the ratchet claw 66 and the ratchet portion 68 is released.

The ratchet release button 65 is provided with separation preventing members 69 and 69 that are unfolded toward a button 65. By deforming the shape of the inner portion of the ratchet release button 65 corresponding to the separation preventing member 69, it is possible the fit the ratchet release button 65 into the through-hole 64 of the pressing members 61 and 61. When the ratchet release button 65 is fitted to the through-hole 64, the separation preventing members 64 are blocked at a stepped portion 64a of the through-hole 64 and thus are not drawn out of the through-hole 64.

In the medical suturing and ligating apparatus according to the third embodiment, as shown in FIG. 3, the loop portion of the ligating wire 18 is hooked onto the root of the pathological lesion portion B while observing the pathological lesion portion B with the endoscope, and the operating wire 9 is made to retract by operating the slider 32. At this time, since the ratchet claw 66 is provided to the slider 32 and the ratchet portion 68 is provided to the operating section body 31, whenever the slider 32 is moved toward the distal end, the ratchet portion 66 engages with the ratchet claw 68 and thus the slider 32 is maintained at the engagement position.

Thereafter, when the slider 32 is fixed and maintained at an appropriate position, the cutting sheath is made to extend by operating the cutting operating section and the ligating wire 18 is cut by the cutting blade 16.

In the third embodiment, the slider 32 can be fixed to the operating section body 31 by engaging the ratchet claw 66 with the ratchet portion 68. Accordingly, it is possible to maintain the state in which the wire holding member 22 is in abutting contact with the distal end of the inner sheath 7 without a need to operate three operating sections at the same time, i.e., without need to operate the slider 32, the operating section body 31, and the cutting operating section 33. Also, it is possible to maintain a predetermined tension applied to the ligating wire 18 until the time of the cutting operation. As a result, it is possible to prevent removal of the wire holding member 22 and thus to cut the ligating wire 18 in a reliable manner.

To disengage the slider 32 from the operating section body 31, it is only necessary to pull in the ratchet release button 65 to move the ratchet claw 66 while resisting the force of the compressing spring 67. In the third embodiment described above, the ratchet claw 66 is provided to the slider 32 and the ratchet portion 68 is provided to the operating section body 31. However, the ratchet claw 66 may be provided to the operating section body 31 and the ratchet portion 68 may be provided to the slider 32.

In the first to third embodiments, the invention has been described for the case of ligating the pathological lesion portion B with the ligating wire 18. However, the invention is also applicable to the case of suturing living tissue, instead of ligating, after the pathological lesion portion is removed.

## Claims

1. A medical suturing and ligating apparatus comprising:
a medical suturing and ligating device comprising:
a suturing and ligating member that is made of a flexible wire and is used for suturing and ligating a living tissue; and
a holding member that holds the suturing and ligating member while a portion of the suturing and ligating member being exposed to the outside;
a flexible sheath to which a proximal end of the suturing and ligating member is inserted;
a cutting member that is provided so as to extend and retract in an axial direction of the flexible sheath and is operable to cut the portion of the suturing and ligating member exposed to the outside from the holding member disposed on the distal end of the flexible sheath when the cutting member is moved to the distal end of the flexible sheath;
an operating section body that is connected to a proximal end of the flexible sheath;
a cutting operating section that is disposed in the vicinity of the operating section body and is connected to the cutting member so as to operate the movement of the cutting member;
a suturing and ligating operating section that is disposed in the vicinity of the operating section body and is connected to the suturing and ligating member so as to operate the suturing and ligating member; and
a sutured or ligated state maintaining device for maintaining a state in which the holding member is in abutting contact with a distal end of the flexible sheath while the suturing and ligating operating section pulls the suturing and ligating member toward the proximal end of the flexible sheath so as to suture or ligate the living tissue with the suturing and ligating member.

2. The medical suturing and ligating apparatus according to claim 1, wherein the sutured or ligated state maintaining device comprises a fixing device for fixing the suturing and ligating operating section at an arbitrary or specific position relative to the operating section body.

3. The medical suturing and ligating apparatus according to claim 2, wherein the fixing device comprises:
a screw that is provided to one of the suturing and ligating operating section and the operating section body; and
an engaging recessed portion that is provided to the other of the suturing and ligating operating section and the operating section body so as to engage the distal end of the screw.

4. The medical suturing and ligating apparatus according to claim 2, wherein the fixing device comprises:
a pin that is provided to one of the suturing and ligating operating section and the operating section body so as to be biased toward the other of the suturing and ligating operating section and the operating section body by a biasing member; and
an engaging recessed portion that is provided to the other of the suturing and ligating operating section and the operating section body so as to engage the distal end of the pin.

5. The medical suturing and ligating apparatus according to claim 2, wherein the fixing device comprises:
a ratchet plate that is provided to one of the suturing and ligating operating section and the operating section body; and
a ratchet claw that is provided to the other of the suturing and ligating operating section and the operating section body so as to engage the ratchet plate and thus to allow a unidirectional movement of the ratchet plate.
